# EUROPEAN PATENT SPECIFICATION

(11) **EP 3 837 511 B1**
(45) Date of publication and mention of the grant of the patent: **07.06.2023**
(21) Application number: 19755815.8
(22) Date of filing: 25.07.2019
(51) Int. Cl.: G01G 19/414, G06Q 10/06

(54) **A SYSTEM AND A PROCESS FOR DELIVERING OPTIMISED MEALS TO PATIENTS**
SYSTEM UND VERFAHREN ZUR LIEFERUNG VON OPTIMIERTEN MAHLZEITEN AN PATIENTEN
SYSTÈME ET PROCÉDÉ PERMETTANT DE DISTRIBUER DES REPAS OPTIMISÉS À DES PATIENTS

(30) Priority: 13.08.2018 EP 18188798
(43) Date of publication of application: 23.06.2021
(73) Proprietor: Blunergy SA, 1015 Lausanne VD (CH)
(72) Inventor: KELLER, Matteo, 6517 Arbedo (CH); KÄUPER, Peter, 1003 Lausanne VD (CH); MOSSIER, Evan, 1004 Lausanne VD (CH); ROSSI, Nathanaël, 1110 Morges VD (CH); SOLIGNAC, Dominique, 1305 Penthalaz (CH)
(74) Representative: Schneiter, Sorin
(86) International application number: PCT/EP2019/070079
(87) International publication number: WO 2020/035282

(56) References cited:
- JP-A- 2005 293 395
- JP-A- 2006 107 238
- US-A1- 2003 091 964
- US-A1- 2004 091 843
- US-A1- 2013 157 233

## Description

### TECHNICAL DOMAIN

The present invention relates generally to the optimisation of a production process, and more particularly to its application in a hospitality environment where optimised control of the preparation of meals for patients is sought, especially where patients' dietary and nutritional requirements and general well-being are to be considered.

### TECHNICAL BACKGROUND

Plate waste is a well-known phenomenon in the health care domain. Techniques are known for measuring food waste through weighing and/or through manual visual estimation of the amount of food remaining on a plate. Studies have shown that up to 65% of prepared food is not consumed in health care institutes, which is a higher level than can be observed in other food service settings. These high levels of food waste can contribute to malnutrition-related complications in hospitals for example and can lead to economic losses. Strategies to minimise waste include reducing portion sizes, providing nutritional fortification additives, using bulk meal delivery systems rather than plate services, providing feeding assistance, providing adequate dining environments and facilities and providing protected meal times.

Food waste systems are known in which a plate of left-over food is weighed, the tare weight is subtracted from the weight of the plate and the left-over food and the weight of the left-over food is calculated and stored. The system also comprises a user interface to allow a user to manually input the types of left-overs that are being weighed and a reason for the food becoming waste. The document US2004/091843 discloses a system for delivering meals to one or more identifiable consumers according to the sate of the art.

### BRIEF DESCRIPTION OF THE INVENTION

In view of the prior art, there is a desire and a need for an economical and comprehensive determination and documentation of the food consumption of the individual patients within a health care unit. There is a need to quantify the weight and type of discarded food and to relate this to the consumption requirements and/or preferences of a particular individual. Furthermore, there is a need to adapt the future preparation of food for the particular individual to optimise the quantity and quality of the food proposed for the individual in terms of his or her nutritional and/or medical requirements and/or his or her personal preferences, thereby providing an opportunity to reduce the amount of discarded food. By nutritional requirements, it is meant its calorific content, its carbohydrate content, protein content, vitamin content, mineral content, salt content, fibre content, fats content, and so on, of the food. By medicinal requirements it is meant pharmacological content of the food or of an additive to the food.

Embodiments of the invention provide for an analysis of the nutritional consumption of patients to be deduced from the observations of food provided compared to observations of food waste or left-overs. This provides for cases of malnutrition to be detected and for meal preparations to be adjusted accordingly to remedy the nutrition content of the meals. Patient studies may also be undertaken using the system and process of the invention in order to observe relationships between nutrition and patient recovery or nutrition in relation to particular pathologies.

According to a first aspect, provision is made for a system for delivering meals to one or more identifiable consumers, each consumer having a pre-determined consumption requirement, comprising:
a database for storing the pre-determined consumption requirements of the identified consumers and for storing a nutrition content and/or calorific content and/or pharmacological content of different ingredients of the meals;
a meal preparation unit configured to prepare an identifiable serving of the meal for the identified consumer based on the pre-determined consumption requirement of the consumer and the nutrition and/or calorific content and/or pharmacological content of different ingredients of the meals according to the database;
an inspection unit for measuring at least one characteristic of the prepared serving of the meal before consumption and for measuring the at least one characteristic of the remains of the meal when the consumer has ceased consumption of the prepared serving;
a consumption monitoring and analysis unit configured to calculate information relative to the consumer's consumption of the prepared serving based on the measurements from the inspection unit and to analyse the information relative to the consumer's consumption of the prepared serving in order to estimate an actual consumption of the meal by the consumer;
characterised in that:
   the characteristic of the prepared serving of the meal is a weight of the prepared serving of the meal and the characteristic of the remains of the meal is a weight of the remains of the meal, the inspection unit comprising an electronic weighing scale configured to weigh the prepared serving of the meal and to weigh the remains of the meal, the consumption monitoring and analysis unit being configured to calculate a difference between the prepared serving of the meal and the remains of the meal based on the measured weights; or
   the characteristic of the prepared serving of the meal is an identification of, or an estimation of a volume of, at least one ingredient of the prepared serving of the meal and a further characteristic of the remains of the meal is an identification of, or an estimation of a volume of, at least one ingredient of the remains of the meal, the inspection unit comprising at least one camera for capturing an image of the prepared serving of the meal and for capturing an image of the remains of the meal, the consumption monitoring and analysis unit being configured to estimate a difference in weight or a difference in volume of the ingredient based on the captured images;
   and in that:
      the monitoring and analysis unit is further configured to update the database with parameters to cause the meal preparation unit to alter a subsequent preparation of a meal for the consumer.

According to a second aspect, there is provided a method for delivering one or more customised meals to one or more identifiable consumers, each consumer having a pre-determined consumption requirement, the method comprising:
preparing a meal for the consumer, the preparation based on a set of pre-determined consumption requirements for the consumer;
during a step of pre-consumption inspection:
   weighing or capturing an image of the ingredients of the prepared meal;
during a step of post-consumption inspection:
   weighing or capturing an image of the left-overs of the meal;
calculating a weight or a volume of consumed food based on the pre-consumption and post-consumption inspection results;
providing instructions for the preparation of a subsequent meal for the consumer, such instructions providing for an alteration in a quantity of an ingredient or for the inclusion of a particular ingredient depending on the result of the calculation of the weight or volume of food consumed.

### BRIEF DESCRIPTION OF THE DRAWINGS

The present invention and its advantages will be better understood with reference to the enclosed drawing and to the detailed description of various embodiments, wherein:

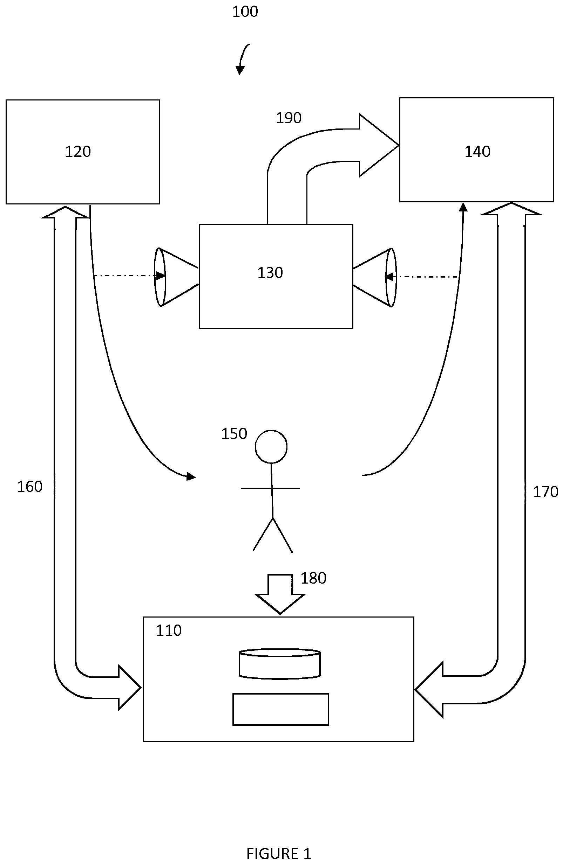

figure 1 illustrates a system in which an embodiment of the present invention may be deployed.

### DETAILED DESCRPTION

Preparation of food for consumers in a health care facility may be achieved through an automated process as in any other commercial food preparation for consumers in other domains such as restaurants and canteens. However, in the domain of health care the food is prepared for a particular consumer or patient, as they are known in the health-care domain. Consequently, a health care unit may have a database to store certain data relative to individual patients in their care, such as age, sex, weight, health status; any particular food allergies, dietary requirements based on medical history, other special dietary requirements (such as those based on religious reasons and socio-economic status) and particular food preferences, for example. According to an embodiment described herein, such data may be used in an automated process for the preparation of a meal for a particular patient. Furthermore, by arranging for the database to be updated with information related to the food actually provided to each patient becomes traceable. Still further, when information is added to the database relative to the amount and type of food left over by the patients, the actual food consumption by each patient also becomes traceable. Analysis of the data related to the food provided to each patient and the food left over by each patient allows for the optimisation of the preparation of the food for each patient and thereby leads to a reduction in the quantity of food left over. Hence, through the use of the process and system described herein, the overall quantity of food prepared and discarded by the health care facility is adjusted, thereby reducing the associated costs.

According to embodiments described herein, an inspection unit is provided for automatically recording particular characteristics related to the food being served and for again automatically recording particular characteristics of the remains of the food once the patient has finished eating. According to a particular embodiment, the inspection unit may comprise a pre-consumption inspection unit and a post-consumption inspection unit. Thus, a separate inspection unit is provided for recording the characteristics of the food when it is prepared and another separate inspection unit is provided for recording the characteristics of the remains of the food. The data thus gathered is analysed, interpreted and fed back into the database.

According to some embodiments, the analysis may be performed for an individual patient. According to other embodiments, the analysis may be performed globally, thus allowing for statistical results to be derived for a particular ward or for the facility in general for example. Similarly, the analysis may allow for studies of food consumption in relation to particular pathologies to be performed, or particular groups of patients, related by age or sex and so on.

Figure 1 illustrates a system (100) in which an embodiment of the present invention may be deployed. The system includes a database (110), a meal preparation unit (120) and an inspection unit (130) at least for inspecting, observing or otherwise documenting the meal that has been prepared for a given patient. The patient is shown in figure 1 with reference number (150), however it is understood that the patient does not form part of the system for delivering meals. According to embodiments of the present invention, the inspection unit is further configured to inspect, observe or otherwise document the remains of the meal after the patient has finished eating and a monitoring and analysis module (140) is configured to receive inspection information from the inspection unit, allowing the monitoring and analysis unit to evaluate a difference between the meal observed at delivery time and the remains of the meal observed at collection time when the patient has finished eating. As mentioned above, the inspection unit may comprise a pre-consumption inspection unit and a post-consumption inspection unit, thus allowing for a first inspection to be carried out close to, or within, the physical location where the food is prepared and a second inspection to be carried out close to, or within, the location where the left-overs, if any, are collected.

A database (110) is provided for the health-care facility. The database may be remote from the health-care facility or located somewhere on-site. The database may store a record for a number of different patients at the health-care facility, comprising an identifier of the patient and a certain amount of the patient's details such as age, sex, weight, medical condition, required food intake per meal or per day in terms of energy requirements, nutritional requirements, dietary requirements. Medical staff may have access to the database, via a user interface (180), to update certain data relating to individual patients should his or her medical condition evolve, resulting in updated food intake requirements in the database for that patient, for example. The database may also store metrics associated with particular foodstuffs used in the ingredients of meals susceptible to be provided for the patients. Such metrics may include, for example, calorific (energy) content per serving amount, or nutritional content per serving amount, such as vitamin content or mineral content, or optimum temperature that a particular foodstuff should be served, and so on. The database may also hold reference images of particular foodstuffs in order to allow for automated estimation of food types and quantities or volumes.

Food is prepared at meal preparation unit (120). For reasons of freshness and for maintaining the food at the required temperature, the meal preparation unit is preferably located within the health-care facility where the patients are located. In other cases, the meal preparation unit may be located at some external facility, especially when adequate provisions are made to maintain the food at the required temperature and to deliver it to the health-care facility quickly. The meal preparation unit has access to the database. According to one embodiment, the meal preparation unit may be a kitchen comprising cooking equipment for preparing different foodstuffs used in the preparation of meal servings for patients. The meal preparation unit may have a computer work station connected to the database via a network connection (160), the workstation being configured to provide instructions, to a cook, for the preparation of personalised meals for each patient based on the patients' records in the database and on the nutritional content and/or the calorific content and/or the pharmacological content of different ingredients of the meals according to the database. The instructions may further be based on a stored list of suggested menus. The workstation then provides the amounts of each ingredient of meals on a patient to patient basis. Either a person prepares the serving of the meal following the instructions provided by the workstation or an automated food dispenser uses the instructions to automatically prepare the meal servings. In order to identify which meal should go to which patient, the servings may be delivered to the relevant patient on a tray which has an electronic identification system such an RFID tag.

According to embodiments of the invention, an inspection unit (130) is provided to inspect the prepared meal to be delivered to a particular patient and to further inspect the remains of the meal at collection once the patient has finished eating. Inspecting in the context of the present invention means recording at least one characteristic of the meal or the remains of the meal. Such a characteristic may be the weight of the meal or of the remains, for example. Another characteristic could be the composition of the meal or of the remains of the meal in terms of the ingredients or in terms of volume.

According to a variant, separate equipment may be provided to inspect the prepared meal and to inspect the remains of the meal. In such a case, it can be said that the inspection unit comprises a pre-consumption inspection unit configured to measure at least one characteristic of the prepared serving of the meal and a separate post-consumption inspection unit configured to measure at least one characteristic of the remains of the meal when the consumer has ceased consumption of the prepared serving.

An embodiment of an inspection unit may comprise a means for identifying the patient for who the meal is intended to be delivered or for identifying the patient who has consumed a meal. An example of such means is an RFID reader to read an RFID tag on a tray on which the prepared meal is placed or on which the remains of the meal are placed. The RFID tag stores an identifier of the patient. Another example is a QR code. The embodiment may further comprise a weighing scale for weighing the prepared meal and/or the remains of the meal. The scale is preferably an electronic scale or at least a scale whose measured value can be converted to an electronic value so that the system can use the measured value. The pre-consumption inspection unit may be configured to verify that the correct meal has been prepared to a particular patient. A check may also be made that the correct ingredients have been used for the meal.

In some embodiments, where it is desirable to estimate the composition of the meal or the remains in terms of their ingredients, the inspection unit may further comprise one or more cameras for capturing one or more images of the meal or the remains of the meal. A multiple-sensor based food item recognition system, for example one which comprises an inter-related plurality of measurement devices and which is configured to use a combination of the measured weights and the images provided by the sensors and to use this to estimate which ingredients are present can be used to more accurately identify and quantify the contents of the meal or the remains. Cameras which are sensitive to different wavelengths of light can be used in order to better identify the ingredients. Infra-red cameras may be used as temperature sensors. Three-dimensional images of the food or the remains may be used to determine the volume of the food or remains.

According to another embodiment, which may be combined with any of the previous embodiments, the inspection unit may further comprise a temperature measurement unit to monitor the temperature of the food upon delivery and/or upon collection since this data may be useful in determining a reason for why a patient might be throwing away all or part of the food delivered.

According to another embodiment, which may be combined with any of the previous embodiments, the inspection unit may further comprise a volumetric determination unit to establish the volume of the food item upon delivery and/or upon collection since this data may be useful in calculating the caloric value of a food item.

According to a particular embodiment, provision is made for further input to be given relative to the appreciation of the meal. For example, as shown in figure 1, provision is made for either a user to input an appreciation (180), for example regarding the volume of the remains or the make-up in terms of ingredients of the remains. The user may be a health-care worker or a person from medical staff or the user may be the patient. The patient may be allowed to input his or her personal preferences, to be taken into consideration for future meal preparation. Alternatively, or in conjunction, the patient may be allowed to input a reason for having either consumed all of the meal or having left some or all of the meal. According to other variants, the input of such appreciations may be provided at another stage such as the monitoring and analysis stages, which will be described later.

An embodiment of a system according to the present invention may further include a consumption monitoring and analysis unit (140), configured to calculate information relative to the consumer's consumption of the prepared serving based on the measurements from the inspection unit or the pre-consumption and post-consumption inspection units. The monitoring and analysis unit may be located on the same premises as the inspection unit or may be located at a remote site, communicably connected (170) to the inspection unit for receiving the inspection data. Indeed, the different actions such as retrieving and adding data to the database, automatically executing mathematical calculations, interpreting data by a trained individual or automatically to come to recommendations to be added into the database are tasks executable in a modern IT environment which may include delocalised computing units or alternatively with discrete computing units linked in a local network.

The monitoring and analysis unit may temporarily store information pertaining to each patient's consumption of the prepared meals and-or the further appreciations described above. The monitoring and analysis unit may also be configured to process and analyse this information, as mentioned before, the monitoring and analysis unit has access to the database. The monitoring and analysis unit may update database entries for different patients depending on the results of the analyses performed. For example, if the result of an analysis shows that a particular patient has finished the whole meal because of an insufficient quantity or has not finished a meal because of a dislike of a certain ingredient or due to the wrong serving temperature of a certain ingredient, then this information may be fed back to the database in order for the preparation of further meals for that particular patient to be altered accordingly.

Health care facilities generally prepare several meals a day for each patient. Systems and methods according to embodiments of the present invention allow for medical requirements and the patient's preferences to be taken into account in the preparation of the food for that individual patient and may rely on data such as the patient's age, sex, weight, health status, food allergies, dietary requirements, religious food requirements, food preferences and the like. The requirements are documented, preferable in electronic form in the database. Furthermore, some health care facilities allow the patients to select a menu or compose their menu individually, within the limited frame of their dietary regime. The menu selections may be documented, preferable in electronic form in the database. The meal preparation unit, having access to the database, can then take this information into account for the preparation of the next upcoming meal for a particular patient. The information may be provided to the preparation unit in hardcopy form or, preferably, in electronic from.

The meals may be prepared and conditioned into individual and identifiable trays at the preparation unit. The tray may be a multi-compartment tray, or one or more plates placed on a serving tray. A meal prepared specifically for an individual patient requires an identifiable and is associated with the particular patient. This may be achieved by a simple piece of paper carrying the patient's name. Preferably however, in order to automate the system, such a paper may carry a QR code or a bar code. Alternatively, any other attachable support other than a paper may be used. For example, an RFID tag may be attached directly onto the tray. Other means may rely on Bluetooth, Wi-Fi or radio frequency transmission and reception.

During or after the food preparation in the preparation unit, different characteristics or metrics of the prepared food may be recorded and transmitted to the monitoring and analysis unit where the characteristics or metrics may be stored along with an identifier of the patient for whom the meal is intended to be delivered. The inspection unit may comprise one or more types of equipment relying on different technologies to record food metrics or characteristics. For example, one or more photos may taken by one or more cameras at the inspection unit after the dish with all food components is prepared. The photos may be transmitted to the monitoring and analysis unit and subsequently analysed in order to interpret their meaning. The database may then be updated depending on the results of the analysis. The photographic representation may be used for purely documentation reasons, providing photographic evidence of the meal and its components prepared for a patient. In an advanced analysis of photographic representations, especially from photos taken from more than one camera, quantification of food components can be executed from photographic data, for example by modelling a 3D representation of the food component and hence determining the weight of the corresponding components or ingredients. Instead of relying on 3D representations built from cameras, radar systems, laser scanners or ultrasound scanners can be used to determine the volume of a food item. If thermal cameras are used, cameras recording wavelengths also in the non-visible infrared part of the spectrum, the temperature of the food components can be determined and documented.

Details regarding the required preparation of a particular patient's meal are communicated from the database to the meal preparation unit, where the relevant menu components may be selected, either by hand or by an automated ingredient selection unit, and placed onto an identifiable tray for the particular patient. As mentioned above, the tray may be rendered identifiable to a particular patient through the use of an RFID tag for example. At the inspection unit, a digital scale may be used to determine the tare of the tray and any other equipment placed thereon, such as cutlery, and subsequently to determine the quantities of individual food components after each addition of the respective food component to the tray, or the removal of the food component as the case may be. Alternatively, the scale may be used to weigh the total weight of the food ingredients or the total weight of the food, the tray and any other items on the tray, such as cutlery. In some embodiments at least one camera may be used to take a photo of the tray as well. RFID data, all determined weights, photos, time stamps of the corresponding metric levy may be sent to the monitoring and analysis unit and stored along with the identifier of the patient. Cutlery and any other non-food items added to the tray may be taken into account in a way which it will not interfere with the weighing and photographing, e.g. by adding such pieces after the weighing and photographing. Alternatively, the captured image or images of the tray may be used to deduce which non-food items were present on the tray and their presence duly taken account of.

As mentioned above, some embodiments may rely on weight information supplied by the inspection unit. For example, a pre-consumption inspection unit may be placed at the food preparation unit. The tray may be weighed without cutlery or glasses etc. The tray may then be weighed with an empty plate for the food in order to obtain the tare weight of the plate. If necessary, the tare weight of tray plus plate plus cutlery may be measured. Following the addition of each component of food to the tray, the weight could be measured again in order to find the weight of the particular component. For example, one component could be potatoes, another component fish and a third component vegetables. According to other embodiments, instead of weight information being used, image information may be used. The image information may further be used to estimate a volume of the food. The volume of the food, given a particular food type, may further be used to calculate the calorific content. According to yet another embodiment, a combination of weight information and image information may be used. Similarly, according to the different embodiments, either weight information, image information or a combination of image and weight information may be used during the post-consumption inspection phase.

Once the meal has been prepared, the prepared meal is then delivered to the patient. In some embodiments, inspection of the prepared meal may be carried out at the meal preparation unit, as described above. In other embodiments, inspection of the prepared meal may be carried out directly at the place where the food is delivered and where the patient will consume it. This has advantages especially when characteristics related to the food inspection include temperature measurements or the time of delivery. An RFID reader may be used at the place where the meal is delivered to the patient to ensure that the patient and the tray delivered to the patient are properly matched. The results of the inspection of the prepared meal may be transmitted to the monitoring and analysis unit using any available transmission means (190) such as Bluetooth or Wi-Fi, where the data may be stored along with the identifier of the patient.

Depending on the services available at a health care facility, after the consumption of the meal the patient may be invited to give his or her appreciation of the menu and the different food ingredients (180). A feedback may cover details regarding the perceived quality and taste as well as sufficiency in terms of the quantity of the meal in general or of certain ingredients or components. The time of delivery of the meal may also be a criterion which is of use in the feedback.

After the patient has finished with his or her meal, a further inspection of the tray is made. This further inspection may be carried out at the premises where the meal was delivered to the patient, in which case the inspection unit may be the same inspection unit which inspected the tray following preparation and before consumption. Alternatively, the food tray may be collected and taken a food discarding and food receptacle cleaning unit of the health care facility. In some cases this may be a part of the same unit which was used for the preparation of the food, in which case the inspection unit may again be the same unit which was used for pre-consumption inspection. Otherwise, if the discarding and cleaning unit is a separate unit, then a further inspection unit may be used to perform a post-consumption inspection of the remnants of the meal following consumption. Any cutlery or other non-food items may either be removed from the tray or automatically taken into consideration as described previously. The RFID information may be read from the tray. At least one digital camera photograph may be taken of the tray, and a digital scale may be used to record or otherwise derive the weight of the tray. A sequenced removal of the food components with intermediate weighing may be executed to recover weight information on the individual remnant food components. RFID data, all determined weights, photos, time stamps of the corresponding metric levy may then be transmitted to the monitoring analysis unit and stored along with the identifier of the patient.

The monitoring and analysis unit may be described as a data collection and interpretation system and may be integrated with an IT system and network of the health care facility. It can be a data base and a software located on a server or it can be a dedicated personal computer.

As mentioned above, analysis of the data may be performed at the monitoring unit. In cases where a separate processing unit is provided for receiving the data from the monitoring unit, this processing unit performs any necessary analysis of the data and may update the database accordingly.

The processing unit may be used to determine the weight of the totality of the food served to a particular patient, or the weight of the individual meal ingredients. Simple mathematical calculations may be used while taking into consideration the tare of the tray. For example, following a first weight measurement of the prepared food and tray, a subtraction of the tare of the tray yields the weight of the added food. In the case of further additions to the tray and weighing, the formerly determined weight is subtracted yielding the weight of the added ingredient. In some embodiments of meal preparation units a system for identifying the type of individual food components added to the tray may include a tactile IT user surface, allowing a quick touch action to identify a food type and thereby allowing for the weight and the type of food component to be determined.

The weight determining procedure is reversed at the discarding unit and again relying on simple mathematical calculations. From the total weight with residual food the tare of tray is subtracted to determine the overall quantity of returned, non-eaten food. In the case of successive removal of food ingredients, after a removal of food component and weighing the tray, this weight is subtracted from the formerly determined weight yielding the weight of the removed ingredient. A system may be put in place for identifying the type of individual food components removed from the tray. An example is a tactile IT user surface at the discarding station allowing a quick touch action to identify a food type and link the weight and the type of food component.

The subtraction of served food minus returned food weight determines the quantity of food eaten by a patient. The subtraction of served food ingredient minus returned food ingredient determines the quantity of consumed individual food components. These values may be used to update the database. Weight to energy (Calorie or Joule) conversion can be performed depending on the calorific value of the menu or the food component and the energy values of food served and consumed may appear as metrics alongside the respective weights and may be used to update the database.

The processing unit may be used to analyse all the gathered data. According to an embodiment, a trained person, such as a medical doctor or a dietitian, may review the food consumption of a patient. Together with the patient's actual and predicted health status development, as determined by the database entries concerning the patient, the food portions, including the calorific values of next meals for the individual patient may be modified and corresponding orders provided to the meal preparation unit in order to deliver the appropriate amount and quality of food to the patient to satisfy his or her culinary tastes and to deliver the appropriate nutritional content and consequently minimise the quantity of food returned for discarding. The trained person may follow the success of their proposed measures over the course of some meals and successively improve the quality and quantity of food preparation.

The aforementioned revising of a patient's food amount and make-up may also be executed by software algorithms analysing actual consumption and predicting future consumption of food by taking into account the patient's personals and health records as recorded in the database.

The aforementioned revising of a patient's food amount may also be monitored by software algorithms analysing actual food consumption and pattern deriving from a typical, expected behaviour can be flagged to a trained person, such as a medical doctor or a dietitian for review. In such an approach, tendencies of malnutrition may be spotted early, and the quality of the health care may be improved.

The elaborated recommendations for a patient's upcoming meal preparations are preferably used to update the database.

All of the embodiments of the invention described herein rely on a comparison of an observation of what is delivered to a patient with an observation of what is returned by the patient. In the embodiments described above, the comparison may use a weight of a tray of food delivered to the patient and a weight of the tray when the patient has finished with the tray of food. In other embodiments the comparison may use an analysis of a captured image of the tray of food which is delivered to the patient and a captured image of the tray once the patient has finished eating. Still other embodiments use a combination of both weights and captured images. In an embodiment described below no weighing is done before the patient receives the food and no images are captured before the patient receives the food. In all other aspects, the features are the same and the inspection unit only inspects the weight and/or the captured images once the patient has finished eating. The comparison is then made with the expected characteristics of the delivered article and the inspected characteristics of the collected article when the patient has finished eating. For example, for a patient A, the database provides the instructions for the preparation of the food. It is therefore already known what the weight of the food should be before delivery. This can be compared with the weight observed by the inspection unit when the patient has finished. Similarly, it is already known what an image of the delivered tray should look like before delivery to the patient. Comparison can be made with one or more images of the remains when the patient has finished. It is known what a volume of each ingredient would be present somewhere on the plate. This can be compared with a volume of food derived by inspection of one or more images of the left-overs.

## Claims

1. A system (100) for delivering meals to one or more identifiable consumers (150), each consumer having a pre-determined consumption requirement, comprising:
a database (110) for storing the pre-determined consumption requirements of the identified consumers and for storing a nutrition content and/or calorific content and/or pharmacological content of different ingredients of the meals;
a meal preparation unit (120) configured to prepare an identifiable serving of the meal for the identified consumer based on the pre-determined consumption requirement of the consumer and the nutrition and/or calorific content and/or pharmacological content of different ingredients of the meals according to the database;
an inspection unit (130) for measuring at least one characteristic of the prepared serving of the meal before consumption and for measuring the at least one characteristic of the remains of the meal when the consumer has ceased consumption of the prepared serving;
a consumption monitoring and analysis unit (140) configured to calculate information relative to the consumer's consumption of the prepared serving based on the measurements from the inspection unit and to analyse the information relative to the consumer's consumption of the prepared serving in order to estimate an actual consumption of the meal by the consumer;
**characterised in that**:
the characteristic of the prepared serving of the meal is a weight of the prepared serving of the meal and the characteristic of the remains of the meal is a weight of the remains of the meal, the inspection unit comprising an electronic weighing scale configured to weigh the prepared serving of the meal and to weigh the remains of the meal, the consumption monitoring and analysis unit being configured to calculate a difference between the prepared serving of the meal and the remains of the meal based on the measured weights; or
the characteristic of the prepared serving of the meal is an identification of, or an estimation of a volume of, at least one ingredient of the prepared serving of the meal and a further characteristic of the remains of the meal is an identification of, or an estimation of a volume of, at least one ingredient of the remains of the meal, the inspection unit comprising at least one camera for capturing an image of the prepared serving of the meal and for capturing an image of the remains of the meal, the consumption monitoring and analysis unit being configured to estimate a difference in weight or a difference in volume of the ingredient based on the captured images;
and **in that**:
the monitoring and analysis unit is further configured to update the database with parameters to cause the meal preparation unit to alter a subsequent preparation of a meal for the consumer.

2. The system according to clam 1, the inspection unit comprising:
an electronic weighing scale configured to weigh the prepared serving of the meal and to weigh the remains of the meal; and
at least one camera for capturing an image of the prepared serving of the meal and for capturing an image of the remains of the meal;
the consumption monitoring and analysis unit being configured to calculate a difference in weight between the prepared serving of the meal and the remains of the meal or a difference in volume based on a combination of the measured weights and the captured images.

3. The system according to any of the preceding claims, wherein the serving of the meal is identifiable and associable with the consumer by way of an RFID tag on a tray on which the serving of the meal is placed.

4. The system according to any of the preceding claims, wherein the inspection unit comprises a separate pre-consumption inspection unit configured to measure the at least one characteristic of the prepared serving of the meal and a separate post-consumption inspection unit configured to measure the at least one characteristic of the remains of the meal when the consumer has ceased consumption of the prepared serving.

5. The system according to any of the preceding claims, wherein a still further characteristic of the prepared serving of the meal is a temperature of the food, the inspection unit comprising a temperature measurement device to measure a temperature of at least a part of the prepared serving of the meal.

6. The system according to any of the preceding claims, wherein the pre-determined consumption requirements of the consumer include a pre-determined minimum and/or a pre-determined maximum consumable intake for the related consumer in terms of a calorific content level and/or a nutritional content level and/or a pharmacological posology level.

7. The system according to any of the preceding claims, wherein the database comprises data relative to the nutritional content of different ingredients for use in the prepared servings of the meals, the preparation unit being configured to use this data in the preparation of the meal serving for the consumer.

8. The system according to any of the preceding claims, wherein the database comprises data relative to the sex, age, weight, health status, food allergies, religion, dietary requirements, or food preferences of the consumer, such data also being used by the preparation unit in the preparation of the meal serving for the consumer.

9. The system according to any of the preceding claims, further comprising a consumer input terminal configured to allow the consumer to input one or more of his or her personal preferences concerning the preparation of a future meal to the monitoring and analysis unit.

10. A method for delivering one or more customised meals to one or more identifiable consumers, each consumer having a pre-determined consumption requirement, comprising:
preparing a meal for the consumer, the preparation based on a set of pre-determined consumption requirements for the consumer;
during a step of pre-consumption inspection:
weighing or capturing an image of the ingredients of the prepared meal;
during a step of post-consumption inspection:
weighing or capturing an image of the left-overs of the meal;
calculating a weight or a volume of consumed food based on the pre-consumption and post-consumption inspection results;
providing instructions for the preparation of a subsequent meal for the consumer, such instructions providing for an alteration in a quantity of an ingredient or for the inclusion of a particular ingredient depending on the result of the calculation of the weight or volume of food consumed.

11. The method according to claim 10, the method comprising, weighing and capturing an image of the ingredients of the prepared meal during a step of pre-consumption inspection and weighing and capturing an image of the left-overs of the meal during a step of post-consumption inspection, the calculation of a weight or volume of consumed food being based on a combination of the captured weights and images.

12. The method according to either claim 10 or claim 11, wherein said preparing of the meal uses at least one ingredient from a set of available ingredients, and the preparation is further based on a reference set of nutrition content, calorific content, and/or pharmacological content of the set of available ingredients.

13. The method according to any of claims 10 to 12, further comprising:
capturing an image of the prepared food during the pre-consumption inspection step;
capturing an image of the remains of the meal during the post-consumption inspection step;
estimating quantities of different ingredients consumed based on a combination of the captured images and the measured weights.

## Patentansprüche

1. System (100) zur Bereitstellung von Mahlzeiten an einen oder mehrere identifizierbare Verbraucher (150), wobei jeder Verbraucher einen vorbestimmten Verzehrbedarfhat, umfassend:
eine Datenbank (110) zum Speichern des vorbestimmten Verzehrbedarfs der identifizierten Verbraucher und zum Speichern eines Nährstoffgehalts und/oder des Kaloriengehalts und/oder des pharmakologischen Gehalts unterschiedlicher Zutaten der Mahlzeiten;
eine Mahlzeitenzubereitungseinheit (120), die derart ausgelegt ist, dass sie eine identifizierbare Portion der Mahlzeit für den identifizierten Verbraucher auf der Grundlage des vorbestimmten Verzehrbedarfs des Verbrauchers und des Nährstoff- und/oder Kaloriengehalts und/oder pharmakologischen Gehalts unterschiedlicher Zutaten der Mahlzeiten gemäß der Datenbank zubereitet;
eine Inspektionseinheit (130) zum Messen wenigstens eines Merkmals der zubereiteten Portion der Mahlzeit vor dem Verzehr und zum Messen des wenigstens einen Merkmals der Reste der Mahlzeit, wenn der Verbraucher den Verzehr der zubereiteten Portion beendet hat;
eine Verzehrüberwachungs- und -analyseeinheit (140), die derart ausgelegt ist, dass sie Informationen über den Verzehr der zubereiteten Portion durch den Verbraucher auf der Grundlage der Messungen der Inspektionseinheit berechnet und die Informationen über den Verzehr der zubereiteten Portion durch den Verbraucher analysiert, um einen tatsächlichen Verzehr der Mahlzeit durch den Verbraucher zu schätzen;
**dadurch gekennzeichnet, dass**:
das Merkmal der zubereiteten Portion der Mahlzeit ein Gewicht der zubereiteten Portion der Mahlzeit ist und das Merkmal der Reste der Mahlzeit ein Gewicht der Reste der Mahlzeit ist, wobei die Inspektionseinheit eine elektronische Waage umfasst, die derart ausgelegt ist, dass sie die zubereitete Portion der Mahlzeit wiegt und die Reste der Mahlzeit wiegt, wobei die Verzehrüberwachungs- und - analyseeinheit derart ausgelegt ist, dass sie eine Differenz zwischen der zubereiteten Portion der Mahlzeit und den Resten der Mahlzeit auf der Grundlage der gemessenen Gewichte berechnet; oder
das Merkmal der zubereiteten Portion der Mahlzeit eine Identifizierung oder eine Schätzung eines Volumens von wenigstens einer Zutat der zubereiteten Portion der Mahlzeit ist und ein weiteres Merkmal der Reste der Mahlzeit eine Identifizierung oder eine Schätzung eines Volumens von wenigstens einer Zutat der Reste der Mahlzeit ist, wobei die Inspektionseinheit wenigstens eine Kamera zum Aufnehmen eines Bildes der zubereiteten Portion der Mahlzeit und zum Aufnehmen eines Bildes der Reste der Mahlzeit umfasst, wobei die Verzehrüberwachungs- und -analyseeinheit derart ausgelegt ist, dass sie einen Gewichtsunterschied oder einen Volumenunterschied der Zutat auf der Grundlage der aufgenommenen Bilder schätzt;
und darin, dass:
die Überwachungs- und Analyseeinheit ferner derart ausgelegt ist, dass sie die Datenbank mit Parametern aktualisiert, um die Mahlzeitenzubereitungseinheit zu veranlassen, eine nachfolgende Zubereitung einer Mahlzeit für den Verbraucher zu ändern.

2. System gemäß Anspruch 1, wobei die Inspektionseinheit umfasst:
eine elektronische Waage, die derart ausgelegt ist, dass sie die zubereitete Portion der Mahlzeit und die Reste der Mahlzeit wiegt; und
wenigstens eine Kamera zum Aufnehmen eines Bildes der zubereiteten Portion der Mahlzeit und zum Aufnehmen eines Bildes der Reste der Mahlzeit;
wobei die Verzehrüberwachungs- und -analyseeinheit derart ausgelegt ist, dass sie eine Gewichtsdifferenz zwischen der zubereiteten Portion der Mahlzeit und den Resten der Mahlzeit oder eine Volumendifferenz auf der Grundlage einer Kombination aus den gemessenen Gewichten und den aufgenommenen Bildern berechnet.

3. System gemäß irgendeinem der voranstehenden Ansprüche, wobei die Portion der Mahlzeit identifizierbar und dem Verbraucher mittels eines RFID-Tags auf einem Tablett, auf dem die Portion der Mahlzeit platziert ist, zuordenbar ist.

4. System gemäß irgendeinem der voranstehenden Ansprüche, wobei die Inspektionseinheit eine separate Inspektionseinheit vor dem Verzehr, die derart ausgelegt ist, dass sie das wenigstens eine Merkmal der zubereiteten Portion der Mahlzeit misst, und eine separate Inspektionseinheit nach dem Verzehr, die derart ausgelegt ist, dass sie das wenigstens eine Merkmal der Reste der Mahlzeit misst, wenn der Verbraucher den Verzehr der zubereiteten Portion beendet hat, umfasst.

5. System gemäß irgendeinem der voranstehenden Ansprüche, wobei ein weiteres Merkmal der zubereiteten Portion der Mahlzeit eine Temperatur des Lebensmittels ist, wobei die Inspektionseinheit eine Temperaturmessvorrichtung umfasst, um eine Temperatur von wenigstens einem Teil der zubereiteten Portion der Mahlzeit zu messen.

6. System gemäß irgendeinem der voranstehenden Ansprüche, wobei der vorbestimmte Verzehrbedarf des Verbrauchers eine vorbestimmte minimale und/oder eine vorbestimmte maximale Verzehrmenge für den betreffenden Verbraucher in Bezug auf den Kaloriengehalt und/oder den Nährstoffgehalt und/oder die pharmakologische Wirkung umfasst.

7. System gemäß irgendeinem der voranstehenden Ansprüche, wobei die Datenbank Daten bezüglich des Nährstoffgehalts unterschiedlicher Zutaten zur Verwendung in den zubereiteten Portionen der Mahlzeiten umfasst, wobei die Zubereitungseinheit derart ausgelegt ist, dass sie diese Daten bei der Zubereitung der Mahlzeitenportion für den Verbraucher verwendet.

8. System gemäß einem der voranstehenden Ansprüche, wobei die Datenbank Daten über das Geschlecht, das Alter, das Gewicht, den Gesundheitszustand, Lebensmittelallergien, die Religion, Diätanforderungen oder Lebensmittelpräferenzen des Verbrauchers enthält, wobei diese Daten auch von der Zubereitungseinheit bei der Zubereitung der Mahlzeitenportion für den Verbraucher verwendet werden.

9. System gemäß irgendeinem der voranstehenden Ansprüche, das weiterhin ein Verbraucher-Eingabeterminal umfasst, das derart ausgelegt ist, dass der Verbraucher eine oder mehrere seiner persönlichen Präferenzen bezüglich der Zubereitung einer zukünftigen Mahlzeit in die Überwachungs- und Analyseeinheit eingeben kann.

10. Verfahren zur Bereitstellung einer oder mehrerer kundenspezifischer Mahlzeiten an einen oder mehrere identifizierbare Verbraucher, wobei jeder Verbraucher einen vorbestimmten Verzehrbedarf hat, umfassend:
Zubereiten einer Mahlzeit für den Verbraucher, wobei die Zubereitung auf einer Reihe von vorher festgelegten Verzehrbedarfen für den Verbraucher basiert;
während eines Inspektionsschritts vor dem Verzehr:
wiegen oder aufnehmen eines Bildes der Zutaten der zubereiteten Mahlzeit;
bei einem Inspektionsschritt nach dem Verzehr:
wiegen oder aufnehmen eines Bildes der Essensreste;
Berechnen eines Gewichts oder eines Volumens der verzehrten Lebensmittel auf der Grundlage der Inspektionsergebnisse vor und nach dem Verzehr;
Bereitstellen von Anweisungen für die Zubereitung einer späteren Mahlzeit für den Verbraucher, wobei diese Anweisungen eine Änderung der Menge einer Zutat oder die Aufnahme einer bestimmten Zutat in Abhängigkeit vom Ergebnis der Berechnung des Gewichts oder Volumens der verzehrten Lebensmittel bereitstellen.

11. Verfahren gemäß Anspruch 10, wobei das Verfahren das Wiegen und Aufnehmen eines Bildes der Zutaten der zubereiteten Mahlzeit während eines Schrittes der Inspektion vor dem Verzehr und das Wiegen und Aufnehmen eines Bildes der Reste der Mahlzeit während eines Schrittes der Inspektion nach dem Verzehr umfasst, wobei die Berechnung eines Gewichts oder Volumens der verzehrten Lebensmittel auf einer Kombination der aufgenommenen Gewichte und Bilder basiert.

12. Verfahren gemäß Anspruch 10 oder Anspruch 11, wobei bei der Zubereitung der Mahlzeit wenigstens eine Zutat aus einer Reihe verfügbarer Zutaten verwendet wird und die Zubereitung ferner auf einer Referenzreihe des Nährstoffgehalts, des Kaloriengehalts und/oder des pharmakologischen Gehalts der Reihe verfügbarer Zutaten basiert.

13. Verfahren gemäß irgendeinem der Ansprüche 10 bis 12, weiterhin umfassend:
Aufnehmen eines Bildes der zubereiteten Lebensmittel während des Inspektionsschritts vor dem Verzehr;
Aufnehmen eines Bildes der Reste der Mahlzeit während des Inspektionsschritts nach dem Verzehr;
Schätzen der Mengen unterschiedlicher verbrauchter Zutaten auf der Grundlage einer Kombination von den aufgenommenen Bildern und den gemessenen Gewichten.

## Revendications

1. Système (100) de livraison de repas à un ou plusieurs consommateurs identifiables (150), chaque consommateur ayant un besoin de consommation prédéterminé, comprenant :
une base de données (110) pour stocker les besoins de consommation prédéterminés des consommateurs identifiés et pour stocker un contenu nutritionnel et / ou un contenu calorifique et / ou un contenu pharmacologique de différents ingrédients des repas ;
une unité de préparation de repas (120) configurée pour préparer une portion identifiable du repas pour le consommateur identifié sur la base du besoin de consommation prédéterminé du consommateur et du contenu nutritionnel et / ou calorifique et / ou pharmacologique de différents ingrédients des repas selon la base de données ;
une unité d'inspection (130) pour mesurer au moins une caractéristique de la portion préparée du repas avant consommation et pour mesurer la au moins une caractéristique des restes du repas lorsque le consommateur a terminé la consommation de la portion préparée ;
une unité de suivi et d'analyse de la consommation (140) configurée pour calculer des informations relatives à la consommation par le consommateur de la portion préparée sur la base des mesures provenant de l'unité d'inspection et pour analyser les informations relatives à la consommation par le consommateur de la portion préparée afin d'estimer une consommation réelle du repas par le consommateur ;
**caractérisé en ce que** :
la caractéristique de la portion préparée du repas est un poids de la portion préparée du repas et la caractéristique des restes du repas est un poids des restes du repas, l'unité d'inspection comprenant une balance électronique configurée pour peser la portion préparée du repas et pour peser les restes du repas, l'unité de suivi et d'analyse de la consommation étant configurée pour calculer une différence entre la portion préparée du repas et les restes du repas sur la base des poids mesurés ; ou
la caractéristique de la portion préparée du repas est une identification de, ou une estimation d'un volume de, au moins un ingrédient de la portion préparée du repas et une autre caractéristique des restes du repas est une identification de, ou une estimation d'un volume de, au moins un ingrédient des restes du repas, l'unité d'inspection comprenant au moins une caméra pour capturer une image de la portion préparée du repas et pour capturer une image des restes du repas, l'unité de surveillance et d'analyse de la consommation étant configurée pour estimer une différence de poids ou une différence de volume de l'ingrédient sur la base des images capturées ;
et **en ce que** :
l'unité de suivi et d'analyse est, en outre, configurée pour mettre à jour la base de données avec des paramètres pour amener l'unité de préparation de repas à modifier une préparation ultérieure d'un repas pour le consommateur.

2. Système selon la revendication 1, l'unité d'inspection comprenant :
une balance électronique configurée pour peser la portion préparée du repas et pour peser les restes du repas ; et
au moins une caméra pour capturer une image de la portion préparée du repas et pour capturer une image des restes du repas ;
l'unité de suivi et d'analyse de la consommation étant configurée pour calculer une différence de poids entre la portion préparée du repas et les restes du repas ou une différence de volume sur la base d'une combinaison des poids mesurés et des images capturées.

3. Système selon l'une quelconque des revendications précédentes, dans lequel la portion du repas est identifiable et associable au consommateur au moyen d'une étiquette RFID sur un plateau sur lequel la portion du repas est placée.

4. Système selon l'une quelconque des revendications précédentes, dans lequel l'unité d'inspection comprend une unité d'inspection pré-consommation distincte configurée pour mesurer ladite au moins une caractéristique de la portion préparée du repas et une unité d'inspection post-consommation distincte configurée pour mesurer ladite au moins une caractéristique des restes du repas lorsque le consommateur a terminé la consommation de la portion préparée.

5. Système selon l'une quelconque des revendications précédentes, dans lequel encore une autre caractéristique de la portion préparée du repas est une température de la nourriture, l'unité d'inspection comprenant un dispositif de mesure de température pour mesurer une température d'au moins une partie de la portion préparée du repas.

6. Système selon l'une quelconque des revendications précédentes, dans lequel les besoins de consommation prédéterminés du consommateur comprennent un apport consommable minimum et / ou maximum prédéterminé pour le consommateur concerné en termes de niveau de contenu calorifique et / ou de niveau de contenu nutritionnel et / ou de niveau de posologie pharmacologique.

7. Système selon l'une quelconque des revendications précédentes, dans lequel la base de données comprend des données relatives au contenu nutritionnel de différents ingrédients à utiliser dans les portions préparées des repas, l'unité de préparation étant configurée pour utiliser ces données dans la préparation de la portion de repas pour le consommateur.

8. Système selon l'une quelconque des revendications précédentes, dans lequel la base de données comprend des données relatives au sexe, à l'âge, au poids, à l'état de santé, aux allergies alimentaires, à la religion, aux exigences diététiques ou aux préférences alimentaires du consommateur, ces données étant également utilisées par l'unité de préparation lors de la préparation de la portion de repas pour le consommateur.

9. Système selon l'une quelconque des revendications précédentes, comprenant en outre un terminal d'entrée consommateur configuré pour permettre au consommateur d'entrer une ou plusieurs de ses préférences personnelles concernant la préparation d'un futur repas dans l'unité de suivi et d'analyse.

10. Procédé pour livrer un ou plusieurs repas personnalisés à un ou plusieurs consommateurs identifiables, chaque consommateur ayant un besoin de consommation prédéterminé, comprenant :
la préparation d'un repas pour le consommateur, la préparation étant basée sur un ensemble d'exigences de consommation prédéterminées pour le consommateur ;
lors d'une étape d'inspection pré-consommation :
la pesée ou la capture d'une image des ingrédients du repas préparé ;
lors d'une étape d'inspection post-consommation :
la pesée ou la capture d'une image des restes du repas ;
le calcul d'un poids ou d'un volume de la nourriture consommée sur la base des résultats d'inspection pré-consommation et post-consommation ;
la fourniture d'instructions pour la préparation d'un repas ultérieur pour le consommateur, ces instructions prévoyant une modification de la quantité d'un ingrédient ou l'inclusion d'un ingrédient particulier en fonction du résultat du calcul du poids ou du volume de la nourriture consommée.

11. Procédé selon la revendication 10, le procédé comprenant la pesée et la capture d'une image des ingrédients du repas préparé pendant une étape d'inspection pré-consommation et la pesée et la capture d'une image des restes du repas pendant une étape d'inspection post-consommation, le calcul d'un poids ou d'un volume de la nourriture consommée étant basé sur une combinaison des poids et des images capturés.

12. Procédé selon la revendication 10 ou la revendication 11, dans lequel ladite préparation du repas utilise au moins un ingrédient d'un ensemble d'ingrédients disponibles, et la préparation est en outre basée sur un ensemble de référence de contenu nutritionnel, de contenu calorifique, et / ou de contenu pharmacologique de l'ensemble d'ingrédients disponibles.

13. Procédé selon l'une quelconque des revendications 10 à 12, comprenant en outre :
la capture d'une image de la nourriture préparée pendant l'étape d'inspection pré-consommation
la capture d'une image des restes du repas pendant l'étape d'inspection post-consommation ;
l'estimation des quantités de différents ingrédients consommés sur la base d'une combinaison des images capturées et des poids mesurés.
